# FASCICULE DE BREVET EUROPEEN

(11) **EP 4 188 320 B1**
(45) Date de publication et mention de la délivrance du brevet: **21.05.2025**
(21) Numéro de dépôt: 21759339.1
(22) Date de dépôt: 29.07.2021
(51) Int. Cl.: A61P 17/02

(54) **COMPOSITION PHOTOPROTECTRICE COMPRENANT UN DERIVE DE GLYCINE BETAÏNE**
LICHTSCHUTZMITTEL ENTHALTEND EIN GLYCIN BETAINE DERIVAT
PHOTOPROTECTIVE COMPOSITION COMPRISING A GLYCINE BETAINE DERIVATIVE

(30) Priorité: 31.07.2020 FR 2008182
(43) Date de publication de la demande: 07.06.2023
(73) Titulaire: Surfactgreen, 60201 Compiegne Cedex (FR)
(72) Inventeur: GALLE, Francis, 35708 RENNES CEDEX 7 (FR); PESSEL, Freddy, 35708 RENNES CEDEX 7 (FR); ROUSSEL, Xavier, 72000 LE MANS (FR)
(74) Mandataire: Renard, Emmanuelle
(86) Numéro de dépôt international: PCT/FR2021/051425
(87) Numéro de publication internationale: WO 2022/023682

(56) Documents cités:
- WO-A1-2005/121294
- FR-A1- 3 013 589
- FABRICE GOURSAUD ET AL: "Glycine betaine as a renewable raw material to "greener" new cationic surfactants", GREEN CHEMISTRY, vol. 10, no. 3, 1 January 2008 (2008-01-01), GB, pages 310, XP055531793, ISSN: 1463-9262, DOI: 10.1039/b713429k
- NNANNA IFENDU A ET AL: "Potential Applications of Protein-Based Surfactants", 1 January 2001, PROTEIN-BASED SURFACTANTS: SYNTHESIS: PHYSICOCHEMICAL PROPERTIES, AND APPLICATIONS, DEKKER, NEW YORK, NY, USA, PAGE(S) 227 - 260, ISBN: 978-0-8247-0004-1, XP008174677

## Description

### OBJET DE L'INVENTION

La présente invention concerne une composition photoprotectrice comprenant, dans un milieu physiologiquement acceptable, au moins un composé photoprotecteur et un tensioactif comprenant au moins un dérivé ester de glycine bétaïne. Elle concerne également cette composition photoprotectrice pour son utilisation dans la protection de la peau contre l'érythème dû aux UVB ou pour son utilisation pour protéger la peau contre certains effets des UVA. Elle concerne enfin l'utilisation du tensioactif précité pour augmenter la résistance au sable d'une composition photoprotectrice.

### ARRIERE-PLAN DE L'INVENTION

En plus d'être responsables de brûlures, les UV sont connus pour être impliqués dans la production de radicaux libres qui entraînent eux-mêmes différentes altérations de la peau, y compris des phénomènes de vieillissement cutané, une sécheresse cutanée, ainsi que la formation de taches pigmentaires et d'irrégularités du teint.

Les compositions photoprotectrices utilisées pour protéger la peau contre les effets des UV se présentent généralement sous la forme d'une émulsion, de type huile-dans-eau ou eau-dans-huile qui contient, à des concentrations diverses, un ou plusieurs filtres organiques lipophiles et/ou des nanopigments minéraux d'oxydes métalliques, capables d'absorber sélectivement les rayonnements UV nocifs, ces filtres et leurs quantités étant sélectionnés en fonction du facteur de protection solaire recherché. Dans de telles émulsions, les filtres hydrophiles sont présents dans la phase aqueuse et les filtres lipophiles (souvent majoritaires) sont présents dans la phase grasse.

Ces émulsions renferment généralement une quantité importante d'huile nécessaire à la solubilisation des filtres UV lipophiles et présentent ainsi souvent un toucher gras et collant susceptible de détourner le consommateur de leur utilisation. Pour cette raison, les émulsions huile-dans-eau sont, d'une manière générale, plus appréciées par le consommateur en raison notamment de leur toucher agréable et de leur présentation sous forme de lait ou de crème non gras. Cependant, elles perdent également plus facilement leur efficacité contre les UV dès lors qu'elles viennent en contact avec l'eau, lors d'une baignade en mer ou en piscine, sous la douche ou lors de la pratique de sports nautiques, dans la mesure où les filtres hydrophiles ont tendance à se dissoudre dans l'eau. Par suite, les compositions solaires qui les contiennent, seuls ou associés aux filtres lipophiles, n'apportent plus la protection initiale recherchée. Par ailleurs, les compositions solaires présentent souvent une mauvaise résistance au sable, ce qui affecte leur efficacité en bord de mer puisque l'utilisateur est découragé de renouveler les applications sur une peau ensablée. Les grains de sable collés sur la peau entraînent également des irritations lors des frottements.

Il serait donc utile de pouvoir disposer de compositions photoprotectrices présentant un toucher non gras et une bonne résistance à l'eau et/ou au sable.

Il a déjà été proposé d'utiliser des agents tensioactifs cationiques pour réduire le toucher gras de compositions de soin de la peau riches en huiles et améliorer la résistance à l'eau, mais également au sable, de compositions photoprotectrices (HAPPI, Décembre 2009, 62-65). Cependant, un certain nombre de ces composés, en particulier les sels d'alkylammonium quaternaires, tels que le chlorure de distéaryldimonium et le chlorure de behentrimonium, ainsi que les amidoquats, tels que le chlorure de palmitamidopropyltrimonium, présentent une biodégradabilité et une écotoxicité insuffisantes. Il existe donc un besoin de mettre au point des compositions photoprotectrices qui soient plus respectueuses de l'environnement.

Parmi les agents cationiques biosourcés, il a déjà été suggéré d'utiliser un esterquat, le chlorure de distéaroyléthyldimonium ou DSEDC (Varisoft^{®} d'EVONIK), dans les compositions de protection de la peau, notamment dans des compositions solaires. Toutefois, ce composé ne peut être formulé qu'en milieu acide et plus précisément à un pH inférieur à 5 et généralement inférieur à 4,5. Or, le pH de la peau saine, qui est déterminé par la constitution du film hydrolipidique présent à sa surface, est en moyenne de 5,5. Il serait donc souhaitable de disposer de composés susceptibles d'être formulés dans une plage plus large de pH et en particulier jusqu'à un pH de 5,5. Par ailleurs, les tensioactifs cationiques précités sont difficilement compatibles avec les additifs anioniques, dont certains sont couramment utilisés comme dispersants de filtres UV, ce qui augmente encore leurs contraintes de formulation.

La Demanderesse a maintenant découvert, de façon inattendue et surprenante, que certains dérivés de glycine bétaïne permettaient d'améliorer la résistance au sable des compositions photoprotectrices.

Il a également été observé que ces composés pouvaient être formulés à un pH allant jusqu'à 5,5 et dans des compositions riches en huile, sans affecter négativement la stabilité ni les propriétés sensorielles de ces compositions.

Ces tensioactifs sont par ailleurs biodégradables et présentent une très faible écotoxicité, qui est même inférieure à celle du DSEDC.

Les demandes de brevet WO 2005/121294, WO 2015/078893 et FR3013589 décrivent des compositions cosmétiques non rincées, notamment des compositions solaires, renfermant des sels d'ester ou d'amide de glycine bétaïne. Dans les deux cas, toutefois, ces dérivés de glycine bétaïne sont présents sous forme de compositions tensioactives renfermant en outre des alkylpolyglycosides éventuellement cationisés.

A la connaissance de la Demanderesse, il n'a toutefois jamais été suggéré d'utiliser aux fins précitées des sels d'ester ou d'amide de glycine bétaïne à chaîne longue, en tant que tels ou sous forme de compositions tensioactives exemptes d'alkylpolyglycosides éventuellement cationisés.

### RESUME DE L'INVENTION

L'invention a pour objet une composition photoprotectrice comprenant, dans un milieu physiologiquement acceptable, au moins un composé photoprotecteur et un tensioactif comprenant au moins un dérivé de glycine bétaïne, étant entendu que ladite composition photoprotectrice ne contient pas d'alkylpolyglycoside éventuellement cationisé,
ledit tensioactif renfermant les constituants suivants :
   (a) au moins un sel d'ester de glycine bétaïne de formule (1) : Xⁿ⁻[(CH₃)₃N⁺-CH₂-COO-R]ₙ,
   (b) au moins un alcool gras de formule R-OH,
   (c) un acide organique ou inorganique de formule XH,
   (d) un sel de glycine bétaïne de formule Xⁿ⁻[(CH₃)₃N⁺-CH₂-COOH]ₙ, et
   (e) au moins un éther de dialkyle de formule R-O-R,
où R est un groupe alkyle linéaire ou ramifié, saturé ou insaturé, comprenant de 8 à 24 atomes de carbone, X est un anion organique ou inorganique et n vaut 1 ou 2.

Elle a également pour objet l'utilisation cosmétique de la composition photoprotectrice mentionnée ci-dessus pour son utilisation dans la protection de la peau contre les effets des UVA choisis parmi : les signes du vieillissement cutané, notamment les rides, le relâchement cutané, la perte de souplesse et/ou d'élasticité de la peau ; la rugosité de la peau ; la perte d'éclat du teint ; les inhomogénéités du teint et notamment les taches pigmentaires ; et/ou le dessèchement de la peau.

Un autre objet de cette invention porte sur une composition photoprotectrice telle que définie ci-dessus, pour son utilisation dans la protection de la peau contre l'érythème dû aux UVB.

Un autre objet encore de la présente invention porte sur l'utilisation d'un tensioactif tel que défini ci-dessus pour augmenter la résistance au sable d'une composition photoprotectrice.

### DESCRIPTION DETAILLEE

La présente invention porte sur une composition photoprotectrice renfermant au moins un dérivé de glycine bétaïne de formule donnée. Par "composition photoprotectrice", on entend une composition renfermant, dans un milieu physiologiquement acceptable, au moins un composé photoprotecteur en quantité suffisante pour bloquer ou absorber les UV, de préférence au moins un filtre UV-A et/ou UV-B organique ou inorganique. Elle confère généralement à la peau une protection contre les UV, déterminée par son Facteur de Protection Solaire, d'au moins 15, voire d'au moins 30, par exemple un Facteur de Protection Solaire de 15, 20, 30 ou 50. Il s'agit d'une composition non rincée. Par "non rincée", on entend que la composition est adaptée et destinée à être appliquée sur la peau et laissée en place au moins une heure, avant d'être éliminée par rinçage à l'eau et/ou à l'aide d'une composition de nettoyage.

Cette composition photoprotectrice renferme un tensioactif à base d'au moins un sel d'ester de glycine bétaïne. Ce dérivé de glycine bétaïne, ainsi que son procédé de préparation seront maintenant décrits plus en détail.

### Dérivés de glycine bétaïne - Sels d'esters de glycine bétaïne

Les sels d'esters de glycine bétaïne peuvent être obtenus suivant un procédé comprenant les étapes successives consistant à :
(1) faire réagir de la glycine bétaïne ou l'un de ses sels avec au moins un alcool gras linéaire ou ramifié, saturé ou insaturé, renfermant de 8 à 24 atomes de carbone, en présence d'un acide organique ou inorganique ;
(2) refroidir le milieu réactionnel à une température de 20 à 90°C ; et
(3) récupérer la composition tensioactive ainsi obtenue.

La première étape de ce procédé consiste à estérifier la glycine bétaïne, ou triméthylglycine. La glycine bétaïne peut être d'origine végétale ou synthétique. Il est nécessaire de la protoner préalablement à l'aide d'un acide organique ou inorganique, dans la mesure où elle se présente sous forme zwitterionique (présence d'une fonction carboxylate). L'acide peut notamment être choisi parmi les acides inorganiques tels que l'acide chlorhydrique, l'acide sulfurique, les acides perhalohydriques, tel que l'acide perchlorique, et leurs mélanges. En variante, il peut être choisi parmi les acides organiques, tels que les acides alkyl sulfuriques, par exemple l'acide décyl ou lauryl sulfurique ; les acides arylsulfoniques, tels que l'acide benzène sulfonique, l'acide paratoluène sulfonique ; les acides alkylsulfoniques, tels que l'acide triflique, l'acide méthanesulfonique, l'acide éthanesulfonique, l'acide décylsulfonique, l'acide laurylsulfonique ou l'acide camphosulfonique ; l'acide sulfosuccinique ; et leurs mélanges. On peut également utiliser des acides de Lewis. De préférence, il s'agit d'un acide alkylsulfonique et en particulier de l'acide méthanesulfonique, dans la mesure où il est facilement biodégradable.

Au cours de l'estérification, la fonction acide de la bétaïne salifiée est mise à réagir avec un alcool gras, pour conduire à un ester de glycine bétaïne sous forme de sel. Par "alcool gras", on entend un alcool linéaire ou ramifié (de préférence linéaire), saturé ou insaturé, comprenant de 8 à 24 atomes de carbone. Des exemples de tels alcools gras peuvent être choisis dans le groupe constitué par : l'octanol (C8:0), le décanol (C10:0), l'undécanol (C11:0), l'alcool laurique (C12:0), l'alcool myristique (C14:0), l'alcool cétylique (C16:0), l'alcool palmitoléique (C16:1), l'alcool stéarique (C18:0), l'alcool oléique (C18:1), l'alcool linoléique (C18:2), l'alcool linolénique (C18:3), l'alcool arachidique (C20:0), l'alcool arachidonique (C20:4), l'alcool béhénique (C22:0), le 2-hexyldécanol, le 2-octyldodécanol, le 2-décyltétradécanol et leurs mélanges. Des mélanges d'alcools gras utilisables peuvent être produits à partir d'une ou plusieurs huiles végétales et notamment d'huile de soja, d'olive, de tournesol, de maïs, de palme, de coprah, de coton, de lin, de germe de blé, de carthame ou de colza, par exemple.

On préfère selon l'invention utiliser un ou plusieurs alcools renfermant de 16 à 22 atomes de carbone et plus préférentiellement un mélange de tels alcools gras.

La réaction d'estérification s'effectue généralement en l'absence de solvant. L'eau produite lors de la réaction contribue par ailleurs à la solubilisation de la glycine bétaïne dans le mélange réactionnel.

Pour la mise en oeuvre de cette réaction, on peut par exemple utiliser de 0,8 à 6,0 équivalents, de préférence de 0,8 à 2 équivalents, par exemple de 0,9 à 1,0 équivalent, ou en variante de 1,1 à 1,8 équivalent, préférentiellement dans ce cas de 1,2 à 1,6 équivalent et, mieux, de 1,3 à 1,5 équivalent d'alcool gras ou dans une seconde variante de 4,0 à 6,0 équivalents, préférentiellement dans ce cas de 4,5 à 5,5 équivalents et, mieux, de 4,8 à 5,2 équivalents d'alcool gras.

On utilise en outre avantageusement de 1,01 à 3,0 équivalents, de préférence de 1,5 à 2,0 équivalents, par exemple de 1,5 à 1,9 équivalent, et préférentiellement de 1,5 à 1,7 équivalent molaire d'acide organique ou inorganique, ou en variante de 1,02 à 1,08 équivalents, préférentiellement dans de cas de 1,03 à 1,07 équivalents et, mieux, de 1,04 à 1,06 équivalent molaire d'acide organique ou inorganique pour 1 équivalent de glycine bétaïne. L'estérification est réalisée à une température allant par exemple de 120 à 180°C, de préférence de 150 à 180°C. La réaction peut être réalisée sous pression atmosphérique ou de préférence sous pression réduite, par exemple à une pression de 10 à 600 mbar. La pression sera généralement d'autant plus faible que la longueur de chaîne de l'alcool gras mis en jeu est grande. Le milieu réactionnel est ensuite refroidi à une température de 20 à 90°C.

On récupère alors la composition tensioactive ainsi obtenue, qui renferme au moins un sel d'ester de glycine bétaïne de formule Xⁿ⁻[(CH₃)₃N⁺-CH₂-COOR]ₙ où : X est un anion organique ou inorganique, R est un radical alkyle correspondant à l'alcool gras R-OH mis en oeuvre dans la réaction d'estérification, et n vaut 1 ou 2.

L'anion X est issu de l'acide mis en oeuvre dans la première étape du procédé et peut donc en particulier être un chlorure, un sulfate, un perchlorate, un ion alkylsulfate, notamment décylsulfate ou laurylsulfate, un ion arylsulfonate, notamment benzène sulfonate, paratoluène sulfonate, un ion alkylsulfonate, notamment triflate, méthanesulfonate, éthanesulfonate, décylsulfonate, laurylsulfonate, camphosulfonate, ou un ion sulfosuccinate. On préfère selon l'invention que X soit choisi parmi les alkylsulfonates et les arylsulfonates, en particulier parmi les ions méthanesulfonate, éthanesulfonate, triflate, paratoluènesulfonate et camphosulfonate. Il s'agit avantageusement de l'ion méthanesulfonate.

Le radical R peut de son côté être choisi parmi les groupements octyle (C8:0), décyle (C10:0), undécyle (C11:0), lauryle (C12:0), myristyle (C14:0), cétyle (C16:0), palmitoléyle (C16:1), stéaryle (C18:0), oléyle (C18:1), linoléyle (C18:2), linolényle (C18:3), arachidyle (C20:0), arachidonyle (C20:4), béhényle (C22:0), 2-hexyldécyle, 2-octyldodécyle et 2-décyltétradécyle.

Il est bien entendu que, dans le cas où plusieurs alcools gras sont mis en oeuvre dans la réaction d'estérification, la composition tensioactive obtenue comprendra plusieurs sels d'esters de glycine bétaïne. L'expression "un sel d'ester de glycine bétaïne" doit donc être comprise, dans le contexte de cette description et sauf indication contraire, comme se référant à un ou plusieurs de ces sels.

Le procédé décrit ci-dessus permet plus précisément d'obtenir une composition tensioactive renfermant les constituants suivants :
(a) au moins un sel d'ester de glycine bétaïne de formule (1) : Xⁿ⁻[(CH₃)₃N⁺-CH₂-COO-R]ₙ,
(b) au moins un alcool gras de formule R-OH,
(c) un acide organique ou inorganique de formule XH,
(d) un sel de glycine bétaïne de formule Xⁿ⁻[(CH₃)₃N⁺-CH₂-COOH]ₙ, et
(e) éventuellement, au moins un éther de dialkyle de formule R-O-R,
où R est un groupe alkyle linéaire ou ramifié, saturé ou insaturé, comprenant de 8 à 24 atomes de carbone, et de préférence de 16 à 22 atomes de carbone, X est un anion organique ou inorganique et n vaut 1 ou 2.

Cette composition tensioactive peut être utilisée telle quelle dans la présente invention. Dans ce cas, elle renferme généralement de 15 à 85 % en poids de sel d'ester de glycine bétaïne.

Il est entendu que, dans le cadre de cette description, on entend par « tensioactif » aussi bien un sel d'ester de glycine bétaïne tel que décrit ici que la composition tensioactive le contenant, obtenue comme décrit précédemment.

Dans une première variante, la composition tensioactive renferme :
(a) de 65 à 85% en poids, de préférence de 70 à 80% en poids, de sel d'ester de glycine bétaïne,
(b) de 1 à 20% en poids, par exemple de 1 à 9% en poids ou de 10 à 20% en poids, d'alcool gras,
(c) de 1 à 20% en poids, par exemple de 5 à 15% en poids d'acide organique ou inorganique,
(d) de 1 à 20% en poids, par exemple de 2 à 15% en poids, de sel de glycine bétaïne,
(e) de 0 à 15 % en poids, par exemple de 2 à 10 % en poids, d'éther de dialkyle.

Dans une seconde variante, la composition tensioactive renferme :
(a) de 15 à 45% en poids, de préférence de 20 à 35%, plus préférentiellement de 25 à 30% en poids, de sel d'ester de glycine bétaïne,
(b) de 50 à 70% en poids, par exemple de 60 à 70% en poids, d'alcool gras,
(c) de 0 à 5% en poids, par exemple de 0 à 1% en poids d'acide organique ou inorganique,
(d) de 0 à 3% en poids, par exemple de 0 à 1% en poids, de sel de glycine bétaïne,
(e) de 0 à 15 % en poids, par exemple de 2 à 10 % en poids, d'éther de dialkyle.

Avantageusement, la composition tensioactive ne renferme pas d'autre constituant que les composants (a) à (e) ci-dessus. En variante, le procédé ci-dessus peut inclure une étape supplémentaire consistant à isoler le sel d'ester de glycine bétaïne présent dans cette composition, qui peut être utilisé en tant que tel dans la présente invention. Dans ce dernier cas, la composition tensioactive utilisée selon l'invention comprendra au moins 90%, de préférence au moins 95%, voire au moins 99% en poids de dérivé de glycine bétaïne.

### Compositions photoprotectrices

Pour la mise en oeuvre de la présente invention, on utilise une composition photoprotectrice renfermant un tensioactif tel que décrit précédemment, de préférence en quantité de 1 à 15% en poids, plus préférentiellement de 2 à 8% en poids, par rapport au poids de la composition photoprotectrice, lorsqu'il s'agit d'une composition tensioactive telle que décrite précédemment. La composition photoprotectrice renferme généralement de 0,5 à 8% en poids, et préférentiellement de 1 à 5% en poids, de dérivé de glycine bétaïne selon l'invention.

La composition photoprotectrice renferme un milieu physiologiquement acceptable, c'est-à-dire compatible avec la peau, en particulier cosmétiquement acceptable, c'est-à-dire qui ne génère pas de picotements ou de rougeurs incompatibles avec une utilisation cosmétique. Ce milieu renferme de préférence une phase aqueuse renfermant de l'eau et/ou de la glycérine et une phase grasse pour former une émulsion. Cette émulsion peut être du type huile-dans-eau (H/E), huile-dans-glycérine, eau-dans-huile (E/H), eau-dans-glycérine ou multiple (par exemple E/H/E). Cette émulsion est préférentiellement du type huile-dans-eau.

La phase aqueuse peut en outre renfermer au moins un gélifiant aqueux. Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar, de tara ou de caroube ; les polymères synthétiques et notamment les homopolymères d'acrylate de sodium éventuellement réticulés, ainsi que les copolymères acryliques, en particulier les copolymères d'acrylate de sodium et/ou de (méth)acrylate d'alkyle et/ou de (méth)acrylate d'hydroxyalkyle et/ou de (méth)acrylate de (polyéthoxy)alkyle, avec éventuellement au moins un autre monomère, avantageusement l'acide 2-acrylamido-2-méthylpropane sulfonique (AMPS), ces copolymères étant éventuellement réticulés ; et leurs mélanges.

De son côté, la phase grasse peut comprendre une ou plusieurs huiles volatiles et/ou non volatiles. Des exemples d'huiles volatiles sont les alcanes ramifiés, tels que l'isododécane, et les alcanes linéaires en C₁₀-C₁₃. Comme huiles non volatiles, on peut citer notamment :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que l'acide linoléique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, de lin, d'argan, d'hibiscus, de coriandre, de pépins de raisin, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse. Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

Dans une forme d'exécution de l'invention, la composition photoprotectrice renferme au moins 20% en poids, de préférence au moins 30% en poids, plus préférentiellement au moins 40% en poids voire au moins 50% en poids d'huile(s). Dans certaines formes d'exécution, la composition photoprotectrice peut même comprendre au moins 60%, par exemple au moins 70%, voire au moins 80% en poids d'huile(s). Il a en effet été observé que les dérivés de glycine bétaïne selon l'invention permettaient de conférer un toucher non gras voire poudré à ces compositions, quand bien même elles renferment un fort taux d'huile(s).

La composition photoprotectrice renferme au moins un filtre UV organique ou inorganique, ou un mélange de ceux-ci, de préférence au moins un filtre UV insoluble. Par filtre UV, on entend tout système capable de filtrer les radiations UVA et/ou UV-B. Par filtre UV insoluble, on entend, au sens de la présente invention, des filtres UV insolubles dans les milieux cosmétiques généralement utilisés dans les formulations solaires et plus particulièrement dont la solubilité dans l'eau à 25°C est inférieure à 0,1 % en poids et dont la solubilité dans l'huile de paraffine à 25°C est inférieure à 1% en poids.

Les filtres UV peuvent être des filtres UV-B (absorption dans la plage de 290 à 320 nm), des filtres UV-A (absorption dans la plage de 320 à 380 nm) ou des filtres à large spectre (absorption dans la plage de 290 à 380 nm). Généralement, on préfère utiliser une combinaison d'au moins deux filtres UV, préférentiellement d'au moins filtre UV-A et d'au moins un filtre UV-B.

Comme exemples de filtres UV organiques, on peut citer ceux qui suivent, désignés par leur nom INCI :
- Butyl methoxydibenzoylmethane ou Avobenzone
- L'acide para-aminobenzoïque (PABA) et ses dérivés tels que Ethyl PABA, Ethyl dihydroxypropyl PABA, Ethylhexyl dimethyl PABA, Glyceryl PABA, PEG-25 PABA
- Les dérivés d'acide salicylique dont : Homosalate, Ethylhexyl salicylate, Dipropylene glycol salicylate, TEA salicylate
- Les dérivés de β,β-diphénylacrylate, tels que Octocrylene, Etocrylene
- Les dérivés de benzophénone, dont Benzophenone-1, Benzophenone-2, Benzophenone-3, Benzophenone-4, Benzophenone-5, Benzophenone-6, Benzophenone-8, Benzophenone-9, Benzophenone-12
- Diethylaminohydroxybenzoylhexyl benzoate ("Uvinul A Plus" de BASF)
- Les dérivés de benzylidènecamphre, tels que 3-Benzylidenecamphor, 4-Methylbenzylidenecamphor ("Eusolex 6300" de Merck), Benzylidenecamphorsulfonic acid, Camphor benzalkonium methosulfate, Terephthalylidenedicamphorsulfonic acid, Polyacrylamidomethylbenzylidenecamphor
- Les dérivés de phénylbenzimidazole, dont Phenylbenzimidazolesulfonic acid ("Eusolex 232" de Merck), Disodium phenyl dibenzimidazole tetrasulfonate
- Les dérivés de phénylbenzotriazole, dont Drometrizole trisiloxane, Methylenebis(benzotriazolyl)tetramethylbutylphenol ("Tinosorb M" de Ciba)
- Les dérivés de triazine, tels que Bis(ethylhexyloxyphenol)methoxyphenyl triazine ("Tinosorb S" de Ciba), Ethylhexyltriazone ( "Uvinul T150" de BASF), Diethylhexylbutamidotriazone ("Uvasorb HEB" de Sigma 3V), la 2,4,6-tris(diisobutyl 4'-aminobenzalmalonate)-s-triazine
- Les dérivés anthraniliques, dont Menthyl anthranilate
- Les dérivés d'imidazoline, tels que Ethylhexyldimethoxybenzylidenedioxoimidazoline propionate
- Les dérivés de benzylmalonate tels que les polyorganosiloxanes contenant des fonctions benzylmalonate, notamment le Polysilicone-15 ("Parsol SLX" de Hoffmann LaRoche)
- Les dérivés de 4,4-diarylbutadiène, dont le 1,1-Dicarboxy(2,2'-dimethylpropyl)-4,4-diphenyl-butadiene
- Les dérivés de benzoxazole, tels que la 2,4-bis[5-(1-dimethylpropyl)benzoxazol-2-yl(4-phenyl)imino]-6-(2-ethylhexyl)imino-1,3,5-triazine ("Uvasorb K2A" de Sigma 3V)
- les dérivés de merocyanine tels que ceux décrits dans WO 2004006878,
- et leurs mélanges.

Les filtres UV sont généralement utilisés dans la composition photoprotectrice selon l'invention en quantité de 0,05% à 30% en poids, de préférence de 5% à 25% en poids, plus préférentiellement de 10 % à 20% en poids, par rapport au poids total de la composition photoprotectrice.

Selon une forme d'exécution préférée, cette composition photoprotectrice renferme en outre au moins un actif anti-âge, en particulier un actif adapté à prévenir et/ou traiter les rides, le relâchement cutané et/ou la formation de taches pigmentaires, qui peut notamment être choisi parmi les agents anti-radicalaires ou anti-oxydants, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique et/ou des fibres élastiques; les agents prévenant la dégradation du collagène et/ou des glycosaminoglycanes et/ou des fibrilles d'ancrage dermo-épidermique et/ou des fibres élastiques ; les agents anti-glycation ; les agents dépigmentants et/ou inhibant la mélanogénèse ; et leurs mélanges.

Des exemples de tels actifs anti-âge sont notamment : l'acide ascorbique, ses sels, ses éthers et ses esters, notamment le glucoside d'ascorbyle ; l'adénosine ; les protéines végétales et leurs hydrolysats ; les polypeptides et les pseudodipeptides ; les silanes tels que le mannuronate de méthylsilanol ; les alpha- et beta-hydroxyacides ; et leurs mélanges.

Selon une forme d'exécution avantageuse de l'invention, la composition photoprotectrice comprend au moins un actif anti-oxydant, qui permet de limiter les dommages oxydatifs engendrés par les UV, notamment des vitamines telles que l'acide ascorbique et ses dérivés et le tocophérol et ses dérivés, l'ubiquinone ou coenzyme Q10, les polyphénols tels que le resvératrol et ses dérivés, ainsi que des extraits de plantes en contenant, notamment des extraits de grenade, de raisin ou de thé vert ; des caroténoïdes tels que le lycopène ainsi que des extraits de plantes en contenant ; et leurs mélanges.

La composition photoprotectrice_selon l'invention peut également renfermer au moins un agent auto-bronzant, tel que la DHA et/ou l'érythrulose.

En variante ou en plus, la composition photoprotectrice selon l'invention peut comprendre au moins un agent hydratant ou humectant, choisi par exemple parmi les polyols tels que le propylène glycol, la glycérine, le pentylène glycol, le xylitol ou le sorbitol ; l'urée ; l'acide hyaluronique et ses sels ; les acides aminés ; et leurs mélanges.

La composition photoprotectrice selon l'invention peut en outre contenir différents constituants qui peuvent être dispersés dans la phase grasse et/ou dans la phase aqueuse qu'elle renferme, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des esters de sucrose, des éthers d'alcools gras et de sucre tels que les alkyl glucosides, et leurs mélanges. Les émulsionnants peuvent représenter de 2 à 10% et de préférence de 4 à 6% du poids total de la composition.

Dans une forme d'exécution de l'invention, la composition photoprotectrice ne renferme pas d'autre émulsionnant que la composition tensioactive décrite précédemment. En particulier, dans le cas des esters de glycine bétaïne, la composition tensioactive les contenant renferme en outre naturellement une quantité plus ou moins importante d'alcool gras qui lui confère des propriétés émulsionnantes remarquables.

La composition photoprotectrice selon l'invention peut en outre comprendre des additifs choisis notamment parmi : des charges pulvérulentes ; des parfums ; des agents séquestrants ; des ajusteurs de pH ; des conservateurs ; des pigments ; des colorants ; et leurs mélanges.

Des exemples d'ajusteurs de pH sont les solutions tampon acide acétique/acétate de sodium et acide succinique/succinate de sodium, le gluconate de sodium et le lactate de sodium. Le pH de la composition photoprotectrice peut être notamment compris entre 2 et 6, de préférence entre 4 et 5,5 et, mieux, entre 5 et 5,5.

La composition photoprotectrice, peut avoir une consistance liquide ou semi-liquide ou une consistance solide. Elle peut se présenter sous toute forme adaptée à une application topique sur la peau et notamment sous forme de lait, de crème, de lotion, ou de gel.

Elle peut notamment être conditionnée dans un tube, un flacon-pompe ou un pot. En variante, elle peut être conditionnée dans un récipient aérosol, afin d'assurer une application de la composition sous forme vaporisée. Dans ce dernier cas, la composition photoprotectrice comprend de préférence au moins un agent propulseur.

### Utilisation

La composition photoprotectrice selon l'invention est adaptée et destinée à être appliqué sur la peau. Par "peau", on entend l'ensemble de la peau du corps à l'exclusion du cuir chevelu et en particulier le visage, le cou, le décolleté, les mains, les bras, les jambes et/ou le ventre.

Elle peut être appliquée sur la peau une ou plusieurs fois par jour , en vue de la protéger contre les effets des UV, en particulier contre l'érythème dû aux UVB et/ou pour prévenir et/ou ralentir l'altération de l'aspect de la peau causée par son exposition aux UVA, en particulier pour prévenir et/ou ralentir les signes du vieillissement cutané, notamment les rides, le relâchement cutané, la perte de souplesse et/ou d'élasticité de la peau ; la rugosité de la peau ; la perte d'éclat du teint ; les inhomogénéités du teint et notamment les taches pigmentaires ; et/ou le dessèchement de la peau.

Le tensioactif selon l'invention permet d'augmenter la résistance au sable de la composition photoprotectrice.

La capacité de résistance au sable des compositions photoprotectrices selon l'invention peut être évaluée en appliquant 0,3g de composition sur l'avant-bras d'un panéliste puis, après une minute, en pulvérisant du sable fin (30 mL) sur la surface traitée. En variante, le protocole d'application modifié décrit dans les exemples ci-après peut être utilisé. Le panéliste tape ensuite trois fois dans ses mains pour éliminer le sable. Le sable restant sur l'avant-bras est alors récupéré par rinçage, séché et pesé.

Le tensioactif selon l'invention peut également augmenter la résistance à l'eau de la composition photoprotectrice. En effet, la charge cationique de ce tensioactif présente une certaine affinité pour la kératine de la peau (globalement chargée négativement), permettant ainsi de former sur la peau un film hydrophobe (grâce aux chaines lipophiles du tensioactif) qui créeune barrière à l'eau.

La résistance à l'eau des compositions photoprotectrices selon l'invention peut être mesurée selon deux méthodes. Dans la première méthode, la résistance à l'eau est évaluée *in vitro* en mesurant l'angle de contact d'une goutte d'eau sur une surface revêtue de la composition photoprotectrice. La seconde méthode consiste à mettre en oeuvre le test in vivo recommandé par le COLIPA dans "Guidelines for Evaluating Sun Product Water Resistance (Décembre 2005)", c'est-à-dire à mesurer le ratio du SPF après action de l'eau par rapport au SPF avant action de l'eau. Le SPF traduit l'augmentation de la durée d'irradiation solaire permise en utilisant la composition. Il correspond au quotient de la durée pour atteindre le seuil érythémateux (dose érythémateuse minimale ou "MED") en présence de la composition photoprotectrice (peau protégée) par rapport à la durée mesurée en l'absence de cette composition (peau non protégée). Le SPF est déterminé in vivo sur peau humaine conformément aux instructions du COLIPA. La MED est la dose la plus faible d'irradiation UV qui, après 16-24h, génère une rougeur légère mais nette de la peau (coup de soleil, érythème). Les sources d'irradiation sont généralement des lampes à xénon.

La composition tensioactive utilisée selon l'invention peut en outre protéger la peau contre les effets cutanés des agressions extérieures. Par « agressions extérieures », on entend notamment des compositions de nettoyage de la peau, plus particulièrement celles contenant au moins un tensioactif anionique, et/ou des facteurs environnementaux tels que des polluants atmosphériques et/ou le rayonnement électromagnétique de longueur d'onde comprise entre 280 et 500 nm, à savoir les UV et la lumière bleue.

Les polluants, mais aussi les UV et la lumière bleue, sont notamment susceptibles d'entraîner une production excessive de radiaux libres responsables de phénomènes de vieillissement cutané. Les principaux polluants atmosphériques qui peuvent avoir un effet délétère sur la peau sont les gaz toxiques (tels que l'ozone, le monoxyde de carbone, les oxydes d'azote ou de soufre), les métaux lourds, présents notamment dans la fumée de cigarette (tels que le mercure, le cadmium ou le plomb), les hydrocarbures polycycliques aromatiques (ou « HPA », tels que le benzopyrène) et des particules fines telles que les PM2.5 qui sont des résidus de combustion sur lesquels une grande quantité de composés organiques et minéraux sont adsorbés. De leur côté, les tensioactifs anioniques, et en particulier les sulfates utilisés dans les produits d'hygiène et d'entretien, ont eux aussi tendance à dessécher la peau. Enfin, les différentes agressions chimiques et physiques auxquelles la peau est soumise modifient en permanence la vitesse et la qualité de renouvellement de l'épiderme. Une altération de la fonction barrière est ainsi observée, qui se manifeste par différents signes visibles tels qu'une peau sèche, la formation de ries superficielles ou plus profondes, un teint terne et/ou l'apparition de taches pigmentaires.

Le tensioactif décrit précédemment peut ainsi permettre de prévenir et/ou ralentir l'altération de l'aspect de la peau causée par les agressions extérieures. Il peut donc également être utilisé dans des compositions cosmétiques non rincées, en particulier des compositions cosmétiques de soin de la peau ou de maquillage (telles qu'un fond de teint).

L'invention sera mieux comprise à la lumière des exemples suivants, qui sont donnés à titre purement illustratif et n'ont pas pour but de limiter la portée de l'invention, définie par les revendications annexées.

### EXEMPLES

### Exemple 1 : Essai de résistance au sable

On a évalué l'effet d'une composition tensioactive selon l'invention sur la résistance au sable d'une composition photoprotectrice.

### 1- Préparation de la composition tensioactive

De la glycine bétaïne (1,0 éq) et un mélange d'alcools gras de C18 à C22 (5,0 éq) sont introduits dans un réacteur. La température de consigne au niveau du mélange est fixée à 150°C et la pression est réduite jusqu'à une valeur de 60 mbar. Une fois les consignes de pression et de température atteintes, une solution d'acide méthanesulfonique 70 % (1,05 éq) est ajoutée au mélange réactionnel. Dès que l'ajout est terminé, la température de consigne est ramenée à 150°C et la pression est maintenue à une valeur de 30 mbar. Six heures après le début de l'introduction de l'acide, on laisse refroidir le mélange réactionnel à 80°C, puis le produit est récupéré, refroidi jusqu'à température ambiante, et constitue la composition tensioactive selon l'invention, qui renferme les constituants suivants :

| | Composition massique |
|---|---|
| Mésylate de bétaïnate d'alkyles en C18-C22 | 25,8% |
| Mésylate de glycine bétaïne | 0,3% |
| Alcools gras en C18-C22 | 65,1% |
| Acide méthanesulfonique | 0,7% |
| Ethers alkyliques en C18-C22 | 8,2% |

### 2- Préparation des compositions photoprotectrices

On a préparé deux compositions photoprotectrices sous forme d'émulsion H/E renfermant les constituants suivants :

| Constituant (nom INCI) | Quantité (% en poids) | |
|---|---|---|
| | Composition A | Composition B |
| HYDROXYETHYLCELLULOSE | 0,5% | 0,5% |
| GLYCERIN | 3,0 % | 3,0 % |
| STEARYL / BEHENYL BETAINATE MESYLATE (AND) STEARYL / BEHENYL ALCOHOL ⁽¹⁾ | 6,0% | |
| BEHENTRIMONIUM CHLORIDE | | 1,98% |
| ARACHIDYL (AND) BEHENYL ALCOHOL | | 4,02% |
| METHYLENE BIS-BENZOTRIAZOLYL TETRAMETHYLBUTYLPHENOL (AND) AQUA (AND) DECYL GLUCOSIDE (AND) PROPYLENE GLYCOL (AND) XANTHAN GUM ⁽²⁾ | 4,0% | 4,0% |
| BUTYL METHOXYDIBENZOYLMETHANE | 3,0% | 3,0% |
| BIS-ETHYLHEXYLOXYPHENOL METHOXYPHENYL TRIAZINE | 6,0% | 6,0% |
| ETHYLHEXYL SALICYLATE | 3,0% | 3,0% |
| C12-15 ALKYL BENZOATE | 6,0% | 6,0% |
| CAPRYLIC / CAPRIC TRIGLYCERIDES | 6,0% | 6,0% |
| BUTYROSPERMUM PARKII BUTTER | 2,0% | 2,0% |
| PERSEA GRATISSIMA (AVOCADO) OIL | 2,0% | 2,0% |
| SODIUM BENZOATE (AND) POTASSIUM SORBATE (AND) AQUA | 0,8% | 0,8% |
| AQUA | qsp 100 % | qsp 100 % |

| | | |
|---|---|---|
| ⁽¹⁾ Composition tensioactive selon l'invention, préparée comme décrit ci-dessus. ⁽²⁾ Parsol Max^{®} de DSM | | |

### 3- Essai de résistance au sable

On a évalué la résistance au sable des Compositions A et B ci-dessus.

Pour ce faire, on a saupoudré 10g de sable sur une surface plane. Parallèlement, on a tracé un rectangle de 7x 4 cm sur l'avant-bras de volontaires, dans lequel 56g de composition A ou B ont ensuite été appliqués (à raison de 2 mg/cm²). On a fait pénétrer la crème par léger massage, puis le bras a été laissé 2 min à l'air libre. L'avant-bras a alors été appliqué sur le sable pendant 10 sec. Après 1 min de pose, on a demandé aux volontaires de taper trois fois dans leurs mains avec la même force. On a ensuite calculé la quantité de sable restée collée sur l'avant-bras.

Trois essais ont été réalisés. La moyenne de ces essais a été calculée et est rapportée dans le tableau ci-dessous :

| | Composition A | Composition B |
|---|---|---|
| Moyenne | 54,87 % | 64,47% |
| Ecart-type | 1,03% | 1,26% |

Il ressort de ces essais que la composition tensioactive selon l'invention confère à la composition photoprotectrice une résistance au sable significativement supérieure à celle d'autres tensioactifs cationiques.

### Exemple 2 : Compositions solaires

Plusieurs types de produits solaires peuvent être préparés à l'aide de compositions tensioactives selon l'invention, ci-après identifiées par GBE, à base de sels d'ester de glycine bétaïne.

Crème solaire solide SPF25 :

| *Matière Première* | *INCI* | *% Matière* |
|---|---|---|
| Tegosoft TN | C12-15 Alkyl Benzoate | 6 |
| Parsol SHIELD | Bis-Ethylhexyloxyphenol Methoxyphenyl Triazine | 6 |
| Parsol 1789 | Butyl Methoxydibenzoylmethane | 3 |
| Parsol Max | Methylene Bis-Benzotriazolyl Tetramethylbutylphenol (and) Aqua (and) Decyl Glucoside (and) Propylene Glycol (and) Xanthan Gum | 4 |
| Parson EHS | Ethylhexyl Salicylate | 3 |
| 1-Octadecanol | Stearyl Alcohol | 20 |
| Myritol 318 | Caprylic/Capric Triglyceride | 10 |
| Beurre de Kokum | Gamicia Indica Seed Butter | 15 |
| AVOCAT HV 1654 | Persea Gratissima (Avocado) Oil | 6 |
| Emogreen L19 | C15-19 Alkane | 8 |
| CosmeGreen ES1822+ | Arachidyl/Behenyl Betainate Mesylate (and) Arachidyl /Behenyl Alcohol | 15 |
| Solution Tampon Lactate/Lactique pH = 4,27 | Sodium Lactate (and) Lactic Acid (and) Aqua | 4 |

Crème solaire Cosmos SPF30 :

| *Matière Première* | *INCI* | *% Matière* |
|---|---|---|
| Cosmos Z75 | Zinc Oxide (and) Dicaprylyl Carbonate (and) Polyhydroxy Stearic Acid (and) Stearic Acid | 18 |
| SFT-85-CC | Titanium Dioxide (and) Silica (and) Jojoba Esters (and) Dicaprylyl Carbonate (and) Polyhydroxystearic Acid | 25 |
| CosmeGreen MB1618 | Cetearyl Betainate Mesylate (and) Cetearyl Alcohol | 15 |
| 1-Octadecanol | Stearyl Alcohol | 20 |
| Emogreen L15 | C15-19 Alkane | 4 |
| Beurre de Karité B1646 | Butyrospernum Parkii Butter | 10 |
| Myritol 318 | Caprylic/Capric Triglyceride | 4 |
| Solution Tampon Lactate/Lactique pH = 4,27 | Sodium Lactate (and) Lactic Acid (and) Aqua | 4 |

## Revendications

1. Composition photoprotectrice comprenant, dans un milieu physiologiquement acceptable, au moins un composé photoprotecteur et un tensioactif comprenant au moins un dérivé de glycine bétaïne, étant entendu que ladite composition photoprotectrice ne contient pas d'alkylpolyglycoside éventuellement cationisé,
ledit tensioactif renfermant les constituants suivants :
(a) au moins un sel d'ester de glycine bétaïne de formule (1) : Xⁿ⁻[(CH₃)₃N⁺-CH₂-COO-R]ₙ,
(b) au moins un alcool gras de formule R-OH,
(c) un acide organique ou inorganique de formule XH,
(d) un sel de glycine bétaïne de formule Xⁿ⁻[(CH₃)₃N⁺-CH₂-COOH]ₙ, et
(e) au moins un éther de dialkyle de formule R-O-R,
où R est un groupe alkyle linéaire ou ramifié, saturé ou insaturé, comprenant de 8 à 24 atomes de carbone, X est un anion organique ou inorganique et n vaut 1 ou 2.

2. Composition photoprotectrice selon la revendication 1, **caractérisée en ce que** le radical R est choisi parmi les groupements octyle (C8:0), décyle (C10:0), undécyle (C11:0), lauryle (C12:0), myristyle (C14:0), cétyle (C16:0), palmitoléyle (C16:1), stéaryle (C18:0), oléyle (C18:1), linoléyle (C18:2), linolényle (C18:3), arachidyle (C20:0), arachidonyle (C20:4), béhényle (C22:0), 2-hexyldécyle, 2-octyldodécyle et 2-décyltétradécyle.

3. Composition photoprotectrice selon la revendication 1 ou 2, **caractérisée en ce que** l'anion X est choisi parmi un chlorure, un sulfate, un perchlorate, un ion alkylsulfate, notamment décylsulfate ou laurylsulfate, un ion arylsulfonate, notamment benzène sulfonate, paratoluène sulfonate, un ion alkylsulfonate, notamment triflate, méthanesulfonate, éthanesulfonate, décylsulfonate, laurylsulfonate, camphosulfonate, ou un ion sulfosuccinate, de préférence parmi les alkylsulfonates et les arylsulfonates, plus particulièrement parmi les ions méthanesulfonate, éthanesulfonate, triflate, paratoluènesulfonate et camphosulfonate, mieux, X est l'ion méthanesulfonate.

4. Composition photoprotectrice selon l'une quelconque des revendications 1 à 3, **caractérisée en ce que** R est un groupe alkyle linéaire ou ramifié, saturé ou insaturé, comprenant de 16 à 22 atomes de carbone.

5. Composition photoprotectrice selon l'une quelconque des revendications 1 à 4, **caractérisée en ce que** le milieu physiologiquement acceptable comprend une phase aqueuse renfermant de l'eau et/ou de la glycérine et une phase grasse pour former une émulsion, de préférence une émulsion du type huile-dans-eau.

6. Composition photoprotectrice telle que définie dans l'une quelconque des revendications 1 à 5, pour son utilisation dans la protection de la peau contre l'érythème dû aux UVB.

7. Composition photoprotectrice selon l'une quelconque des revendications 1 à 5 pour son utilisation dans la protection de la peau contre les effets des UVA choisis parmi : les signes du vieillissement cutané, notamment les rides, le relâchement cutané, la perte de souplesse et/ou d'élasticité de la peau ; la rugosité de la peau ; la perte d'éclat du teint ; les inhomogénéités du teint et notamment les taches pigmentaires ; et/ou le dessèchement de la peau.

8. Composition photoprotectrice selon la revendication 7, **caractérisée en ce qu'**elle est appliquée sur le visage, le cou, le décolleté, les mains, les bras, les jambes et/ou le ventre.

9. Utilisation d'un tensioactif tel que défini dans l'une quelconque des revendications 1 à 5 pour augmenter la résistance au sable d'une composition photoprotectrice.

## Patentansprüche

1. Lichtschutzzusammensetzung, die in einem physiologisch annehmbaren Medium mindestens eine Lichtschutzverbindung und ein Tensid umfasst, das mindestens ein Glycinbetainderivat umfasst, unter der Bedingung, dass die Lichtschutzzusammensetzung kein gegebenenfalls kationisiertes Alkylpolyglycosid enthält, wobei das Tensid die folgenden Bestandteile enthält:
(a) mindestens ein Glycinbetainestersalz der Formel (1): Xⁿ⁻[(CH₃)₃N⁺-CH₂-COO-R]ₙ,
(b) mindestens einen Fettalkohol der Formel R-OH,
(c) eine organische oder anorganische Säure der Formel XH,
(d) ein Glycinbetainsalz der Formel Xⁿ⁻[(CH₃)₃N⁺-CH₂-COOH]ₙ und
(e) mindestens einen Dialkylether der Formel R-O-R,
wobei R eine gesättigte oder ungesättigte, geradkettige oder verzweigte Alkylgruppe ist, die 8 bis 24 Kohlenstoffatome umfasst, X ein organisches oder anorganisches Anion ist und n 1 oder 2 beträgt.

2. Lichtschutzzusammensetzung nach Anspruch 1, **dadurch gekennzeichnet, dass** der Rest R aus den Gruppen Octyl (C8:0), Decyl (C10:0), Undecyl (C11:0), Lauryl (C12:0), Myristyl (C14:0), Cetyl (C16:0), Palmitoleyl (C16:1), Stearyl (C18:0), Oleyl (C18:1), Linoleyl (C18:2), Linolenyl (C18:3), Arachidyl (C20:0), Arachidonyl (C20:4), Behenyl (C22:0), 2-Hexyldecyl, 2-Octyldodecyl und 2-Decyltetradecyl ausgewählt ist.

3. Lichtschutzzusammensetzung nach Anspruch 1 oder 2, **dadurch gekennzeichnet, dass** das Anion X aus einem Chlorid, einem Sulfat, einem Perchlorat, einem Alkylsulfat-, insbesondere Decylsulfat- oder Laurylsulfation, einem Arylsulfonat-, insbesondere Benzolsulfonat-, Paratoluolsulfonation, einem Alkylsulfonat-, insbesondere Triflat-, Methansulfonat-, Ethansulfonat-, Decylsulfonat-, Laurylsulfonat-, Camphersulfonation oder einem Sulfosuccination, vorzugsweise aus Alkylsulfonaten und Arylsulfonaten, noch spezieller aus Methansulfonat-, Ethansulfonat-, Triflat-, Paratoluolsulfonat- und Camphersulfonationen ausgewählt ist, X noch mehr bevorzugt das Methansulfonation ist.

4. Lichtschutzzusammensetzung nach einem der Ansprüche 1 bis 3, **dadurch gekennzeichnet, dass** R eine gesättigte oder ungesättigte, geradkettige oder verzweigte Alkylgruppe ist, die 16 bis 22 Kohlenstoffatome umfasst.

5. Lichtschutzzusammensetzung nach einem der Ansprüche 1 bis 4, **dadurch gekennzeichnet, dass** das physiologisch annehmbare Medium eine Wasser und/oder Glycerin enthaltende wässrige Phase und eine Fettphase umfasst, um eine Emulsion, vorzugsweise eine Emulsion vom Öl-in-Wasser-Typ, zu bilden.

6. Lichtschutzzusammensetzung gemäß der Definition in einem der Ansprüche 1 bis 5 zur Verwendung beim Schutz der Haut vor einem UVB-bedingten Erythem.

7. Lichtschutzzusammensetzung nach einem der Ansprüche 1 bis 5 zur Verwendung beim Schutz der Haut vor den Wirkungen von UVA, ausgewählt aus: Symptomen der Hautalterung, insbesondere Falten, der Hauterschlaffung, dem Verlust der Geschmeidigkeit und/oder der Elastizität der Haut; der Rauheit der Haut; dem Verlust des strahlenden Teints; Inhomogenitäten des Teints und insbesondere Pigmentflecken und/oder dem Austrocknen der Haut.

8. Lichtschutzzusammensetzung nach Anspruch 7, **dadurch gekennzeichnet, dass** sie auf das Gesicht, den Hals, das Dekolleté, die Hände, die Arme, die Beine und/oder den Bauch aufgetragen wird.

9. Verwendung eines Tensids gemäß der Definition in einem der Ansprüche 1 bis 5 zur Erhöhung der Beständigkeit einer Lichtschutzzusammensetzung gegen Sand.

## Claims

1. A photoprotective composition comprising, in a physiologically acceptable medium, at least one photoprotective compound and a surfactant comprising at least one glycine betaine derivative, it being understood that said photoprotective composition does not contain optionally cationized alkylpolyglycoside,
said surfactant including the following constituents:
(a) at least one glycine betaine ester salt of formula (1): Xⁿ⁻[(CH₃)₃N⁺-CH₂-COO-R]ₙ,
(b) at least one fatty alcohol of formula R-OH,
(c) an organic or inorganic acid of formula HX,
(d) a glycine betaine salt of formula Xⁿ⁻[(CH₃)₃N⁺-CH₂-COOH]ₙ, and
(e) at least one dialkyl ether of formula R-O-R,
where R is a saturated or unsaturated, linear or branched, alkyl group comprising from 8 to 24 carbon atoms, X is an organic or inorganic anion and n has the value 1 or 2.

2. The photoprotective composition as claimed in claim 1, **characterized in that** the radical R is chosen from the octyl (C8:0), decyl (C10:0), undecyl (C11:0), lauryl (C12:0), myristyl (C14:0), cetyl (C16:0), palmitoleyl (C16:1), stearyl (C18:0), oleyl (C18:1), linoleyl (C18:2), linolenyl (C18:3), arachidyl (C20:0), arachidonyl (C20:4), behenyl (C22:0), 2-hexyldecyl, 2-octyldodecyl and 2-decyltetradecyl groups.

3. The photoprotective composition as claimed in claim 1 or 2, **characterized in that** the anion X is chosen from a chloride, a sulfate, a perchlorate, an alkyl sulfate ion, in particular a decyl sulfate or lauryl sulfate ion, an arylsulfonate ion, in particular a benzenesulfonate or para-toluenesulfonate ion, an alkylsulfonate ion, in particular a triflate, methanesulfonate, ethanesulfonate, decylsulfonate, laurylsulfonate or camphorsulfonate ion, or a sulfosuccinate ion, preferably from the alkylsulfonates and the arylsulfonates, more particularly from the methanesulfonate, ethanesulfonate, triflate, para-toluenesulfonate and camphorsulfonate ions; better still, X is the methanesulfonate ion.

4. The photoprotective composition as claimed in any one of claims 1 to 3, **characterized in that** R is a saturated or unsaturated, linear or branched, alkyl group comprising from 16 to 22 carbon atoms.

5. Photoprotective composition according to any one of claims 1 to 4, **characterized in that** the physiologically acceptable medium comprises an aqueous phase containing water and/or glycerine and a fatty phase to form an emulsion, preferably an emulsion of the oil-in-water type.

6. The photoprotective composition as defined in any one of claims 1 to 5, for its use in the protection of the skin against erythema due to UVB rays.

7. Composition as claimed in any one of claims 1 to 5 for its use in protecting the skin against the effects of UVA rays chosen from: signs of skin aging, in particular wrinkles, sagging of the skin, loss of suppleness and/or of elasticity of the skin; roughness of the skin; loss of radiance of the complexion; nonuniformities of the complexion and in particular pigment spots; and/or drying of the skin.

8. Photoprotective composition as claimed in claim 7, **characterized in that** it is applied to the face, neck, neckline, hands, arms, legs and/or stomach.

9. The use of a surfactant as defined in any one of claims 1 to 5 for increasing the resistance to sand of a photoprotective composition.
